# EUROPEAN PATENT APPLICATION

(11) **EP 3 069 715 A1**
(43) Date of publication of application: **21.09.2016**
(21) Application number: 15160172.1
(22) Date of filing: 20.03.2015
(51) Int. Cl.: A61K 9/20, A61K 31/135

(54) **Immediate release dosage forms of Atomoxetine**

(71) Applicant: Salmon Pharma GmbH, 4002 Basel (CH)
(72) Inventor: AMMER,RICHARD, 4002 Basel (CH)
(74) Representative: Drescher, Christian

(57) **Abstract**

The invention relates to a plurality of immediate release pharmaceutical dosage forms comprising Atomoxetine in different amounts and at least one pharmaceutically acceptable excipient. The amount of Atomoxetine ranges from 10mg to 100mg and each dosage form comprises the same qualitative composition. Furthermore, the strengths represent either a combination of two homologous series of weight proportional dosage forms or the combination of a series of weight proportional dosage forms with a series of dose proportional dosage forms.

## Description

### INTRODUCTION

Atomoxetine is a drug that is approved for treatment of attention deficit / hyperactivity disorder (ADHD).

Chemically Atomoxetine is (3R)-N-methyl-3-(2-methylphenoxy)-3-phenylpropan-1-amine, the compound having the following structure:

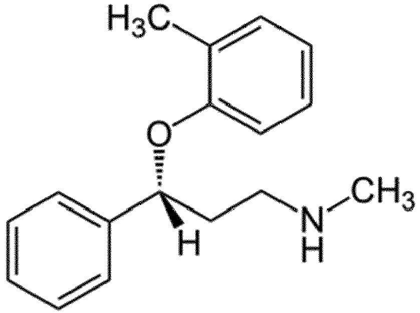

Atomoxetine is marketed in the form of its hydrochloride salt under the brand name Strattera^{®}.

Strattera^{®} is marketed in 7 different strengths, i.e. 10mg, 18mg, 25mg, 40mg, 60mg, 80mg or 100mg hard capsules in Europe and the United States of America.

A synthesis of Atomoxetine hydrochloride is disclosed in EP 0 052 492.

### DEFINITIONS

The term "Bioequivalence Guideline" as used in the present application shall refer to the Guideline on the investigation of bioequivalence, issued 20 January 2010 by the European Medicines Agency (EMA).

Unless indicated otherwise, the term "weight proportional" as used in the present application shall be understood as being synonymous to "quantitative proportional" according to the Bioequivalence Guideline.

Unless indicated otherwise, the term "dose proportional" for two compositions shall indicate that only the amount of a filler is changed to account for the change in amount of active substance, the amounts of other core excipients or capsule content being the same for the concerned strengths, in accordance with the Bioequivalence Guideline c) i. and ii. or c) i. and iii.

Unless indicated otherwise, the terms "Immediate release dosage form", "strength", "active substance", "excipient", "core excipient", "capsule content" and "qualitative composition" as used in the present application shall be understood as defined in the Bioequivalence Guideline.

The term "ingredient" as used in the present application shall be understood as to comprise active substance and excipients

The term "Atomoxetine" as used in the present application shall refer to Atomoxetine base, whereas the term "Atomoxetine HCl" shall refer to the hydrochloride salt of Atomoxetine, i.e. Atomoxetine hydrochloride.

The term "pre-blend" as used in the present application shall be understood as a mixture of an active substance with one or more excipient(s) which may be divided up into several portions (i.e. strengths) with different total quantities, but the same qualitative composition.

Unless indicated otherwise, all amounts in percent (%) or weight-% shall be understood as indicating amounts in weight-% relative to the total weight of the respective composition.

### DESCRIPTION OF THE INVENTION

In order obtain a market authorization for a generic drug development, several regulatory requirements have to be met. Amongst these requirements, the proof of bioequivalence is of major importance. Bioequivalence is generally proven by appropriate bioequivalence studies.

The total weight of Strattera^{®} 10mg, 18mg, 25mg and 40mg capsules is 280mg, the total weight of Strattera^{®} 60mg and 80mg capsules is 370mg and the total weight of Strattera^{®} 100mg capsules is 450mg. Obviously, the standard approach, i.e. the development of "1:1"- copies of Strattera^{®} capsules in all marketed strengths would inevitably result in the need of at least 7 bioequivalence studies and would thus bear the disadvantages of high costs and a high risk of failure.

The Bioequivalence Guideline offers the opportunity to reduce the number of strengths to be investigated via bioequivalence studies. The guideline requires several "general biowaiver criteria" to be met, but does not provide any guidance on how to apply these criteria to any specific product. In particular, there is no guidance on how to develop oral dosage forms of Atomoxetine in 7 different strengths with the aim to reduce the number of costly bioequivalence studies.

According to the Bioequivalence Guideline, bioequivalence has to be demonstrated at the strength(s) that are most sensitive to detect a potential difference between products. In vivo bioequivalence studies for the other strength(s) may be waived only if the following general biowaiver criteria are met:
a) the pharmaceutical products are manufactured by the same manufacturing process,
b) the qualitative composition of the different strengths is the same,
c) the composition of the strengths are quantitatively proportional, i.e. the ratio between the amount of each excipient to the amount of active substance(s) is the same for all strengths (for immediate release products coating components, capsule shell, color agents and flavors are not required to follow this rule),
   If there is some deviation from quantitatively proportional composition, condition c is still considered fulfilled if condition i. and ii. or i. and iii. below apply to the strength used in the bioequivalence study and the strength(s) for which a waiver is considered
   i. the amount of the active substance(s) is less than 5 % of the tablet core weight/ the weight of the capsule content
   ii. the amounts of the different core excipients or capsule content are the same for the concerned strengths and only the amount of active substance is changed
   iii. the amount of a filler is changed to account for the change in amount of active substance. The amounts of other core excipients or capsule content should be the same for the concerned strengths
d) appropriate in vitro dissolution data should confirm the adequacy of waiving additional in-vivo bioequivalence testing.

According to the Bioequivalence Guideline, the bioequivalence study should generally be carried out with the highest strength. In the case of Atomoxetine, the highest strength contains 100mg Atomoxetine.

The application of the Bioequivalence Guideline for Atomoxetine is connected with several difficulties. Facing the fact that the amount of Atomoxetine in Strattera^{®} varies by a factor of 10 between the highest (100mg) and the lowest (10mg) strength, applying a simple approach of quantitatively proportional strengths is unfavorable:
In the case that the highest strength (100mg Atomoxetine) has a total weight of 450mg (as does the corresponding brand product Strattera^{®}), the lowest strength (10mg Atomoxetine) has only a total weight of 45mg. This is not only much less compared to the 280mg weight of the corresponding brand product Strattera^{®}, but also causes problems in the technical handling as well as compliance problems for the patients because the formulation has a very low weight and is small in size. Generally it is accepted that a pharmaceutical dosage form shall exhibit a minimum total weight of 100mg.

If, on the other hand, the lowest strength (10mg Atomoxetine) has a total weight of 280mg as does the corresponding brand product Strattera^{®}, the highest strength (100mg Atomoxetine) would exhibit a total weight of 2,800mg. This is also not favorable since it would not only exceed the weight of the corresponding brand product Strattera^{®} (450mg) by far, but also causes problems in the technical handling as well as compliance problems for the patients because of its heavy weight and huge size. On the contrary, it is desired that none of the strengths of a generic version of Strattera^{®}should exceed a total weight of the original, i.e. 450mg.

If there is some deviation from quantitatively proportional compositions, the Bioequivalence Guideline offers several alternatives (see c) i., ii. and iii.). However, also the simple application of these alternatives gives rise to several drawbacks:
If the highest strength with 100mg Atomoxetine had an Atomoxetine-content of below 5% by weight, thus satisfying biowaiver criterion c) i., and only the amount of active substance is changed in order to satisfy biowaiver criterion c) ii., the lowest strength with 10mg Atomoxetine would only comprise about 0.499% by weight of active substance.

If the lowest strength with 10mg Atomoxetine had a maximum Atomoxetine-content of below 5% by weight, thus satisfying biowaiver criterion c) i., and only the amount of filler is changed in order to satisfy biowaiver criterion c) iii., the total weight of a tablet of the highest strength with 100mg Atomoxetine would exhibit a total weight of at least 2,333mg. Such a finished product would not only exceed the weight of the corresponding brand product Strattera^{®} (450mg) by far, but also cause heavy compliance problems for the patients, rendering this approach unfeasible.

In summary, there is no guidance in the Bioequivalence Guideline how to develop generic Atomoxetine compositions without the need of up to 7 bioequivalence studies and the preparation of 7 pre-blends, thus without envisaging severe drawbacks from commercial and/or patient compliance point of view.

It has now been found that it is indeed possible to design generic Atomoxetine compositions in all 7 currently marketed strengths which do not suffer from any of the disadvantages expressed above.

A first feasible approach is the combination of two homologous series of weight proportional formulations. By combining one set of 4 weight proportional dose dosage forms with a set of another 3 weight proportional dose dosage forms (table 1 & 2) or combining of one set of 5 weight proportional dose dosage forms with a set of another 2 weight proportional dose dosage forms (table 3), the following advantages are achieved:
- the number of clinical studies may be reduced from 7 down to only 2 in-vivo bioequivalence studies, which reduces the costs for those studies and reduces the risk of failure of those studies significantly
- the 7 individual dosage forms may be prepared from only 2 different pre-blends, thus allowing more flexibility in splitting one pre-blend into different strengths
- None of the strengths exceeds the total weight of the original brand product Strattera^{®}, i.e. 450mg
- The total tablet weight of all strengths is within a preferred range from the technical point of view, i.e. is at least 100mg

Total weights and Atomoxetine hydrochloride content of equally advantageous pharmaceutical compositions are displayed in tables 1 to 3 below:

**Table 1**

| **Strength** | **Total weight of composition** | **Atomoxetine HCl content [weigth %]** |
|---|---|---|
| | | |
| 100mg | 166mg - 450mg | 25.40% - 68.86% |
| 80mg | 133mg - 360mg | |
| 60mg | 100mg - 270mg | |
| | | |
| 40mg | 400mg - 450mg | 10.16%- 11.43% |
| 25mg | 250mg - 281.25mg | |
| 18mg | 180mg - 202.50mg | |
| 10mg | 100mg - 112.50mg | |

**Table 2**

| Strength | Total weight of composition | Atomoxetine HCl content [weigth %] |
|---|---|---|
| | | |
| 100mg | 250mg - 450mg | 25.4% - 45.72% |
| 80mg | 200mg - 360mg | |
| 60mg | 150mg - 270mg | |
| 40mg | 100mg - 180mg | |
| | | |
| 25mg | 250mg - 450mg | 6.35% - 11.43% |
| 18mg | 180mg - 324mg | |
| 10mg | 100mg - 180mg | |

**Table 3**

| **Strength** | **Totam weigth of composition** | **Atomoxetine HCl content [weigth%]** |
|---|---|---|
| | | |
| 100mg | 400mg - 450mg | 25.4% - 28.58% |
| 80mg | 320mg - 360mg | |
| 60mg | 240mg - 270mg | |
| 40mg | 160mg - 180mg | |
| 25mg | 100mg - 112.5mg | |
| | | |
| 18mg | 180mg - 450mg | 4.57% - 11.43% |
| 10mg | 100mg - 250mg | |

A second feasible approach is the combination of one homologous series of 5 weight proportional dose dosage forms comprising from 25mg up to 100mg Atomoxetine with one series of 2 dose proportional dosage forms comprising from 10mg up to 18mg Atomoxetine on the basis of Biowaiver criteria c) i. and ii., where the amount of a filler is changed to account for the change in amount of active substance whereas the amounts of other core excipients or capsule content remain the same for the concerned strength. With pharmaceutical compositions exhibiting the total weights and Atomoxetine hydrochloride contents as displayed in table 4 below, the following advantages are achieved:
- the number of clinical studies may be reduced from 7 down to only 1 in-vivo bioequivalence study, thus reducing both costs and risk of failure of those studies to a minimum
- the 7 individual dosage forms may be prepared from only 3 different pre-blends
- All strengths submitted to a dose proportional approach satisfy the requirement of a total content of Atomoxetine hydrochloride of less than 5% by weight, thus satisfying Bioequivalence criterion c)i.
- None of the strengths exceeds the total weight of the original brand product Strattera^{®}, i.e. 450mg
- The total tablet weight of all strengths is within a preferred range from the technical point of view, i.e. is at least 100mg

**Table 4**

| **Strength** | **Total weight of composition** | **Atomoxetine HCl content [weight %]** |
|---|---|---|
| 100mg | 412.0mg - 450.0mg | 25.4% - 28.58% |
| 80mg | 329.6mg - 360.0mg | |
| 60mg | 247.2mg - 270.0mg | |
| 40mg | 164.8mg - 180.0mg | |
| 25mg | 103.0mg - 112.5mg | |
| 18mg | 412.0mg - 450.0mg¹⁾ | 4.58% - 4.99% |
| 10mg | 412.0mg - 450.0mg¹⁾ | 2.77% - 2.54% |

| | | |
|---|---|---|
| ¹⁾ The total weight must be identical to the total weight of the 100mg reference strength | | |

A similar approach on the basis of Biowaiver criterion c) iii.) cannot be applied because when the changed amount of active substance is not replaced by a filler, the total content of Atomoxetine hydrochloride in the 18mg strength exceeds 5% by weight and Biowaiver criterion c) i. is not fulfilled.

The immediate release dosage forms of the compositions according to the present invention include, but are not limited to oral dosage forms such as capsules or tablets.

According to the patient information leaflet, Strattera^{®} capsules must not be opened because the capsule content comprising the active drug can irritate the eye. In the event of the contents of the capsules coming into contact with the eye, the affected eye should be flushed immediately with water, and medical advice obtained. Hands and any other part of the body that may have come into contact with the capsule contents should also be washed as soon as possible. In addition, US 2006-0057099 reports of an extreme caustic and bitter taste of the Atomoxetine in Strattera^{®}. By using a pharmaceutical formulation of a film coated tablet the possible release of irritating content as in case of the opening of a capsule can be avoided and the bitter taste of Atomoxetine is easily masked by means of the film coating. Accordingly, in a particular preferred embodiment of the invention the finished product is in form of tablets, preferably film coated.

The pharmaceutical formulations according to the present invention may be manufactured according to processes well known in the art.

In the following, the terms fillers, disintegrants, binders, lubricants, glidants etc. used shall be understood as including a single compound, but also mixtures of compounds. Pharmaceutically acceptable fillers include dicalcium phosphate, (pregetatinized) starch, lactose, calcium carbonate, magnesium carbonate, sorbitol, mannitol, sucrose, dextrine, kaolin, magnesium oxide, calcium sulfate, xylitol, isomalt, glucose, fructose, maltose, acids like citric acid, tartaric acid, fumaric acid, co-polymers such as those from vinyl pyrrolidone and vinyl acetate or those of polyethylene glycol, and mixtures thereof. A particular preferred filler for tablet formulations is dicalcium phosphate. Dicalcium phosphate is advantageous because of its good compressibility, taken into account that the relative amount of filler varies by a factor of about 100% and there is no possibility to compensate any change of any property by adapting even the quantitative amount of any other excipient in order to satisfy certain biowaiver criteria of the guidelines.

Pharmaceutically acceptable disintegrants include croscarmellose sodium, sodium starch glycolate, crospovidone, and mixtures thereof. Preferred disintegrant is croscarmellose sodium.

Pharmaceutically acceptable lubricants include magnesium stearate, calcium stearate, dimethicone, stearic acid, glyceryl behenate, hexanedioic acid, hygrogenated vegetable oil, sodium stearyl fumarate, glycerine fumarate, and mixtures thereof. Preferred lubricant is magnesium stearate.

Pharmaceutically acceptable binders include microcrystalline cellulose, povidone, hydroxypropyl methylcellulose, hydroxy propylcellulose, sodium carboxyl methyl cellulose, and mixtures thereof. Preferred binder is microcrystalline cellulose.

Pharmaceutically acceptable glidants include tricalcium phosphate, colloidal silicon dioxide, and mixtures thereof. Preferred glidant is tricalcium phosphate.

Pharmaceutically acceptable coatings include typical ready-to-use coating mixtures comprising polymer, plasticizer and pigments.

The tablets according to the invention may comprise further pharmaceutically acceptable excipients.

Preferred tablets according to the present invention comprise:

**Table 5**

| **Ingredient (tablet core)** | **Function** | **Range [weigth %]** |
|---|---|---|
| Atomoxetine HCl | Active substance | 2.5 - 68.9 |
| Dicalcium phosphate (DCPA) | Filler | 25 - 60 |
| (e.g. DICAFOS A-150^{®}) | | |
| Microcrystalline Cellulose (MCC) | Binder | 25 - 40 |
| (e.g. VIVAPUR 12^{®}] | | |
| Tricalcium phosphate (TCP) | Glidant | 2 - 10 |
| (e.g. TRICAFOS 500^{®}) | | |
| Croscarmellose | Disintegrant | 1 - 5 |
| (e.g. AcDiSol^{®}) | | |
| Mg-Stearate | Lubricant | 0.5 - 3 |
| (e.g. LIGAMED MF-2-V^{®}) | | |

| **Coating** | | |
|---|---|---|
| OPADRY II^{®} 85 F 48 105 | Coating | 0 - 3 |

The particle size of the Atomoxetine hydrochloride ranges from 50µm to 500µm, preferably from 200µm to 400µm (D₉₀-value), being measured according to standard light scattering techniques.

The tablet cores of the present invention may be manufactured via standard processes well known in the art.

A typical formulation process of tablet cores comprises the following steps:
(a) The drug substance, fitter(s), binder(s), glidant(s) and portions of disintegrant(s) are weighed, sieved (0.80mm - 1.0mm mesh width) and mixed to form mixture I
(b) lubricant(s) and portion of disintegrant are weighed, sieved (0.5mm mesh width), combined with mixture I and mixed to form the final blend
(c) the final blend is compressed into tablets
(d) the tablets are finally film coated

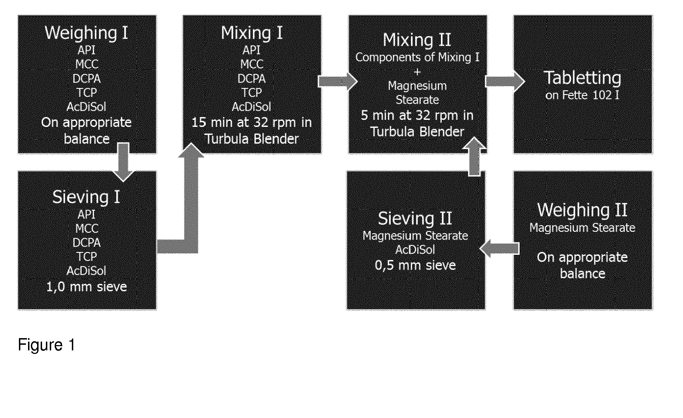

A schematic overview of the tableting process according to the present invention is displayed in figure 1. The tablet cores obtained this way may optionally be film coated by coating techniques well known in the art. For parameters of the film coating procedure see Example 6.

### EXAMPLES

### Example 1 (Reference) - Composition of Strattera^{®}

**Table 6**

| **Strength** | **100mg** | **80mg** | **60mg** | **40mg** | **25mg** | **18mg** | **10mg** |
|---|---|---|---|---|---|---|---|
| Atomoxetine hydrochloride | 114.3mg | 91.44mg | 68.58mg | 45.72mg | 28.58mg | 20.58mg | 11.43mg |
| Total weight Strattera® [mg] | 450mg | 370mg | 370mg | 280mg | 280mg | 280mg | 280mg |
| Relative drug content [weight %] | 25.4% | 24.7% | 18.5% | 16.3% | 10.2% | 7.35% | 4.08% |

As can be seen in table 6 above, the relative drug content of each strength of Strattera^{®} is different. If a 1:1 generic copy of Strattera^{®} is intended, inter alia matching the exact total weight of each strength, 7 BE-studies have to be performed since none of the biowaiver criteria of the Bioequivalence Guideline can be met.

### Example 2 - Combination of two homologous series of weight proportional dosage forms

**Table 7**

| **Strength** | | **100mg** | **80mg** | **60mg** | | **40mg** | **25 mg** | **18mg** | **10mg** |
|---|---|---|---|---|---|---|---|---|---|
| Atomoxetin HCl | | 114.30mg | 91.44mg | 68.58mg | | 45.72mg | 28.58mg | 20.58mg | 11.43mg |
| Dicalcium phosphate | | 114.66mg | 91.73mg | 68.80mg | | 183.18mg | 114.49mg | 82.43mg | 45.80mg |
| Microcrystalline Cellulose | | 155.40mg | 124.73mg | 93.24mg | | 155.40mg | 97.13mg | 69.93mg | 38.85mg |
| Tricalcium phosphate | | 23.10mg | 18.48mg | 13.86mg | | 23.10mg | 14.44mg | 10.40mg | 5.78mg |
| Croscarmellose | | 8.40mg | 6.72mg | 5.04mg | | 8.40mg | 5.25mg | 3.78mg | 2.10mg |
| Mg-Stearate | | 4.20mg | 3.36mg | 2.52mg | | 4.20mg | 2.63mg | 1.89mg | 1.05mg |
| Tablet core weight | | 420mg | 336mg | 252mg | | 420mg | 262.5mg | 189.00mg | 105.00mg |

The 80mg and 60mg strengths are quantitatively proportional with respect to the 100mg first reference strength, thus satisfying biowaiver criterion c) of the Bioequivalence Guideline. The same applies for the 25mg, 18mg and 10mg strengths with respect to the 40mg second reference strength.

Accordingly, only 2 in-vivo bioequivalence studies with the 100mg and 25mg strength are necessary, whereas the other strengths can be waived. The 7 individual dosage forms may be prepared from only 2 pre-blends.

### Example 3 - Combination of two homologous series of weight proportional dosage forms

**Table 8**

| **Strength** | | **100mg** | **80mg** | **60mg** | **40mg** | | **25mg** | **18mg** | **10mg** |
|---|---|---|---|---|---|---|---|---|---|
| Atomoxetin HCl | | 114.30mg | 91.44mg | 68.58mg | 45.72mg | | 28.58mg | 20.58mg | 11.43mg |
| Dicalcium phosphate | | 114.66mg | 91.73mg | 68.80mg | 45.86mg | | 200.32mg | 144.23mg | 80.13mg |
| Microcrystalline Cellulose | | 155.40mg | 124.73mg | 93.24mg | 62.16mg | | 155.40mg g | 111.89mg | 62.16mg |
| Tricalcium phosphate | | 23.10mg | 18.48mg | 13.86mg | 9.24mg | | 23.10mg | 16.63mg | 9.24mg |
| Croscarmellose | | 8.40mg | 6.72mg | 5.04mg | 3.36mg | | 8.40mg | 6.05mg | 3.36mg |
| Mg-Stearate | | 4.20mg | 3.36mg | 2.52mg | 1.68mg | | 4.20mg | 3.02 mg | 1.68mg |
| Tablet core weight | | 420mg | 336mg | 252mg | 168 mg | | 420mg | 302.4mg | 168,00mg |

The 80mg, 60mg and 40mg strengths are quantitatively proportional with respect to the 100mg first reference strength, thus satisfying biowaiver criterion c) of the Bioequivalence Guideline. The same applies for the 18mg and 10mg strength with respect to the 25mg second reference strength.

Accordingly, only 2 in-vivo bioequivalence studies with the 100mg and 25mg strength are necessary, whereas the other strengths can be waived. The 7 individual dosage forms may be prepared from only 2 pre-blends.

### Example 4 - Combination of two homologous series of weight proportional dosage forms

**Table 9**

| **Strength** | | **100mg** | **80mg** | **60mg** | **40mg** | **25mg** | | **18mg** | **10mg** |
|---|---|---|---|---|---|---|---|---|---|
| Atomoxetin HCl | | 114.30mg | 91.44mg | 68.58mg | 45.72mg | 28.58mg | | 20.58mg | 11.43mg |
| Dicalcium phosphate | | 114.66mg | 91.73mg | 68.80mg | 45.86mg | 28.67mg | | 208.32mg | 115.73mg |
| Microcrystallin Cellulose e | | 155.40mg | 124.73mg | 93.24mg | 62.16mg | 38.85mg | | 155.40mg | 86.33mg |
| Tricalcium phosphate | | 23.10mg | 18.48mg | 13.86mg | 9.24mg | 5.78mg | | 23.10mg | 12.83mg |
| Croscarmellose | | 8.40mg | 6.72mg | 5.04mg | 3.36mg | 2.10mg | | 8.40mg | 4.67mg |
| Mg-Stearate | | 4.20mg | 3.36mg | 2.52mg | 1.68mg | 1.05mg | | 4.20mg | 2.33mg |
| Tablet core weight | | 420mg | 336mg | 252mg | 168 mg | 105mg | | 420mg | 233.33mg |

The 80mg, 60mg, 40 and 25mg strengths are quantitatively proportional with respect to the 100mg first reference strength, thus satisfying biowaiver criterion c) of the Bioequivalence Guideline. The same applies for the 10mg strength with respect to the 18mg second reference strength.

Accordingly, only 2 in-vivo bioequivalence studies with the 100mg and 25mg strength are necessary, whereas the other strengths can be waived. The 7 individual dosage forms may be prepared from only 2 pre-blends.

### Example 5 - Combination of one homologous series of weight proportional dosage forms with a series of dose proportional dosage forms

**Table 10**

| **Strength** | | **100mg** | **80mg** | **60mg** | **40mg** | **25mg** | | **18mg** | **10mg** |
|---|---|---|---|---|---|---|---|---|---|
| Atomoxetin HCl | | 114.30mg | 91.44mg | 68.58mg | 45.72mg | 28.58mg | | 20.58mg | 11.43mg |
| Dicalcium phosphate | | 114.66mg | 91.73mg | 68.80mg | 45.86mg | 28.67mg | | 208.32mg | 217.47mg |
| Microcrystallin Cellulose e | | 155.40mg | 124.73mg | 93.24mg | 62.16mg | 38.85mg | | 155.40mg | 155.40mg |
| Tricalcium phosphate | | 23.10mg | 18.48mg | 13.86mg | 9.24mg | 5.78mg | | 23.10mg | 23.10mg |
| Croscarmellose | | 8.40mg | 6.72mg | 5.04mg | 3.36mg | 2.10mg | | 8.40mg | 8.40mg |
| Mg-Stearate | | 4.20mg | 3.36mg | 2.52mg | 1.68mg | 1.05mg | | 4.20mg | 4.20mg |
| Tablet core weight | | 420mg | 336mg | 252mg | 168 mg | 105mg | | 420mg | 420mg |

The 100mg, 80mg, 60mg, 40mg and 25mg strengths are quantitatively proportional, thus satisfying biowaiver criterion c) of the Bioequivalence Guideline.

Both the 10mg and the 18mg strength contain less than 5% of active substance, thus satisfying biowaiver criteria c) i. of the Bioequivalence Guideline.

The 10mg strength is dose proportional to the 18mg strength in the way that only the amount of a filler is changed to account for the change in amount of active substance compared to the strength used in the bioequivalence study, thus satisfying biowaiver criterion c) iii. of the Bioequivalence Guideline.

Accordingly, only two in-vivo bioequivalence studies with the 100mg and 18mg strength are necessary whereas the other strengths can be waived.

The 7 individual dosage forms may be prepared from only 3 different pre-blends.

### Example 6 - Coating procedure

The coating was performed in a perforated drum coater and a common parameter setting as recommended by the supplier of the mentioned excipient. A weight gain of between 2.5% and 3.5% was considered being optimal in order to ensure a complete coating. The main parameters during the coating procedure are summarized in table 11.

**Table 11**

| Strength | Weight gain | Inlet temperature | Exhaust temperature | Product temperature |
|---|---|---|---|---|
| 10mg | 3.2 % | 62°C | 48-51°C | 44-48°C |
| 18mg | 2.9 % | 62-65°C | 47-50°C | 43-51°C |
| 25mg | 3.5 % | 60°C | 49-51°C | 45-50°C |
| 40mg | 3.5 % | 62°C | 49°C | 45-49°C |
| 60mg | 3.4 % | 62-63°C | 48-51°C | 43-49°C |
| 80mg | 2.8 % | 63-65°C | 50°C | 44-48°C |
| 100mg | 2.9 % | 62-65°C | 47-50°C | 43-45°C |

### Example 7 - Dissolution characteristics

The in-vitro dissolution of all samples according to the inventions as well as Strattera^{®} capsules were analyzed according to Ph. Eur. 2.9.3 - Apparatus 1 (Basket) with the settings as displayed in table 12.

**Table 12**

| | |
|---|---|
| Media: | pH 1.2: HCl 0.1N |
| | pH 4.5: Acetate buffer |
| | pH 6.8: Phosphate buffer |
| Volume: | 500ml |
| Temperature: | 37.0°C ± 0.5°C |
| Stirring speed: | 75 rpm |

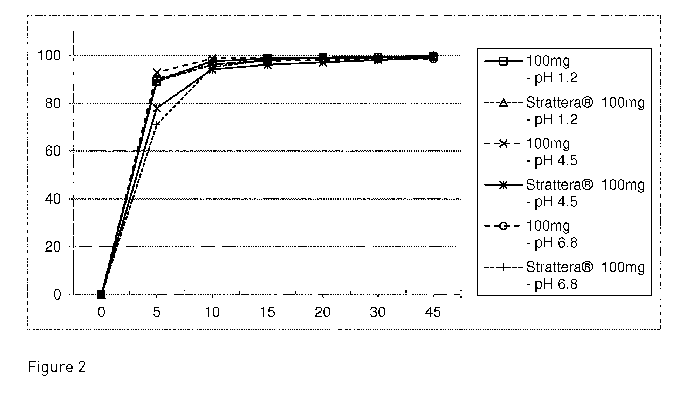

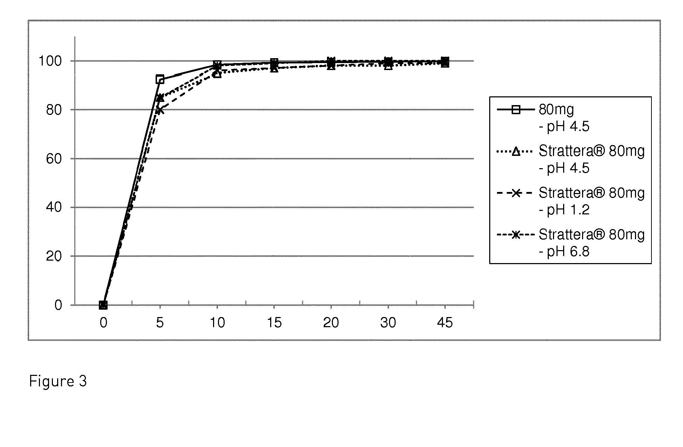

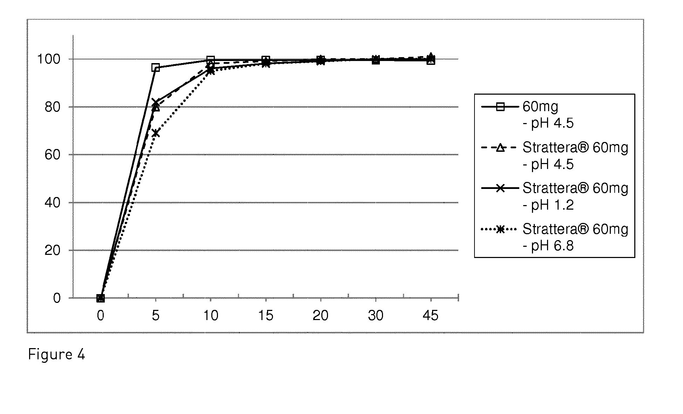

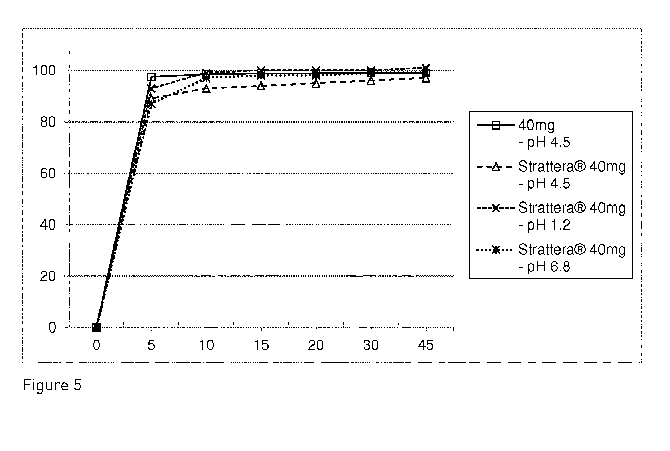

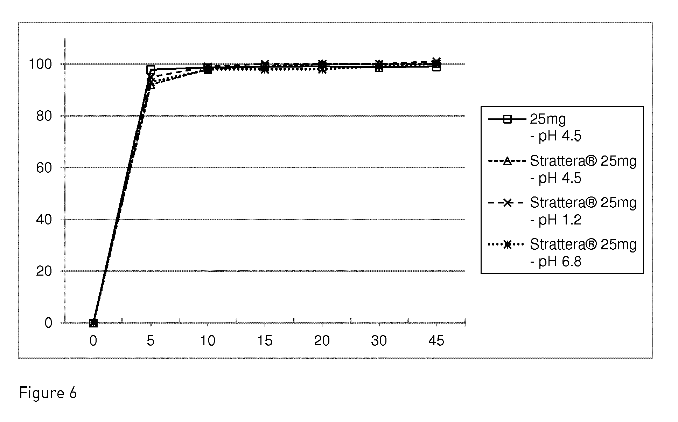

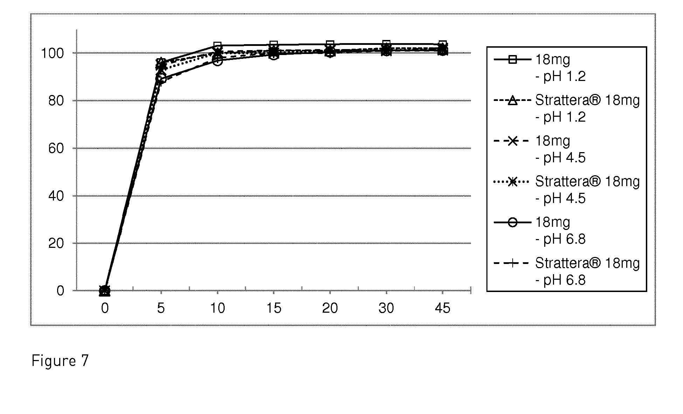

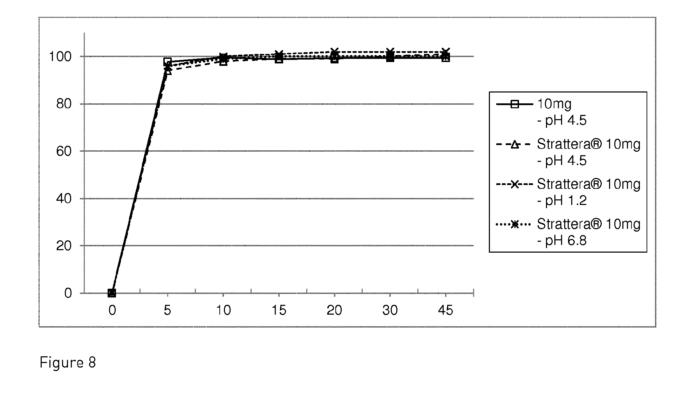

The in-vitro dissolution profiles of formulations according to Example 5 of the present invention in comparison to the original Strattera^{®} are displayed in figures 2 to 8 at different pH-values.
Figure 2: 100mg film coated tablets (n=6) 100mg Strattera^{®} (n=12)
Figure 3 : 80mg film coated tablets (n=6); 80mg Strattera^{®} (n=12)
Figure 4: 60mg film coated tablets (n=6); 60mg Strattera^{®} (n=12)
Figure 5: 50mg film coated tablets (n=6); 40mg Strattera^{®} (n=12)
Figure 6: 25mg film coated tablets (n=6); 25mg Strattera^{®} (n=12)
Figure 7: 18mg film coated tablets (n=6); 18mg Strattera^{®} (n=12)
Figure 8: 10mg film coated tablets (n=6); 10mg Strattera^{®} (n=12)

Values on the x-axis represent time in minutes, whereas values on the y-axis represent the percentage of dissolution.

The comparisons of all in-vitro dissolution profiles with those of the brand market product Strattera^{®} show a very similar dissolution profile for all strengths at all pH-values, i.e. at pH 1.2, pH 4.5 and pH 6.8.

## Claims

1. A plurality of immediate release pharmaceutical dosage forms comprising Atomoxetine or one of its pharmaceutically acceptable salts as active substance in different amounts and one or more pharmaceutically acceptable excipient(s); wherein
- the amount of Atomoxetine present in each dosage form defines the strength of the dosage form and ranges from 10mg to 100mg;
- each dosage form comprises the same qualitative composition;
- each strength of a first set of dosage forms comprising one or more strengths is weight proportional compared to a first reference strength;
- each strength of a second set of dosage forms comprising one or more strengths is dose proportional compared to a second reference strength, said dosage forms of the second set containing less than 5% by weight of active substance compared to the total weight of the compositions of the dosage forms; and
- the first and the second reference strength are the same or different.

2. The plurality of dosage forms according to claim 1 in form of tablets or capsules,
wherein
- at least one pharmaceutically acceptable excipient is a filler
- the first and the second reference strength are identical and comprise 100mg Atomoxetine;
- dosage forms comprising from 25mg to 80mg Atomoxetine are weight proportional compared to the reference strength; and
- dosage forms comprising from 10mg to 18mg Atomoxetine are dose proportional compared to the reference strength in the way that only the amount of a filler is changed to account for the change in amount of active substance whereas the amounts of other core excipients or capsule content are the same for the concerned strengths.

3. The plurality of dosage forms according to claim 1 or claim 2, wherein the active substance is Atomoxetine in the form of its hydrochloride salt.

4. The plurality of dosage forms according to claim 3, wherein
- the strengths are 10mg, 18mg, 25mg, 40mg, 60mg, 80mg and 100mg;
- the 100mg, the 18mg and the 10mg strength exhibit the same total weight in a range from 412mg to 450mg;
- the 80mg strength exhibits a total weight in a range from 329.6mg to 360.0mg;
- the 60mg strength exhibits a total weight in a range from 247.2mg to 270.0mg;
- the 40mg strength exhibits a total weight in a range from 164.8mg to 180.0mg; and
- the 25mg strength exhibits a total weight in a range from 103.0mg to 112.5mg.

5. A plurality of immediate release pharmaceutical dosage forms comprising Atomoxetine or one of its pharmaceutically acceptable salts as active substance in different amounts and one or more pharmaceutically acceptable excipient(s); wherein
- the amount of Atomoxetine present in each dosage form defines the strength of the dosage form and ranges from 10mg to 100mg;
- each dosage form comprises the same qualitative composition;
- each strength of a first set of dosage forms comprising one or more strengths is weight proportional compared to a first reference strength;
- each strength of a second set of dosage forms comprising one or more strengths is weight proportional compared to the second reference strength.

6. A plurality of 7 dosage forms according to claim 5, wherein
- the first reference strength comprises 100mg Atomoxetine;
- dosage forms comprising from 60mg to 80mg Atomoxetine are weight proportional compared to the first reference strength;
- the second reference strength comprises 40mg Atomoxetine; and
- the dosage form comprising from 10mg to 25mg Atomoxetine are weight proportional compared to the second reference strength.

7. The plurality of dosage forms according to claim 6, wherein
- the strengths are 10mg, 18mg, 25mg, 40mg, 60mg, 80mg and 100mg;
- the active substance is Atomoxetine in the form of its hydrochloride salt;
- the 100mg strength exhibits the same total weight of from 166mg to 450mg;
- the 80mg strength exhibits a total weight of from 133mg to 360mg;
- the 60mg strength exhibits a total weight of from 100mg to 270mg;
- the 40mg strength exhibits a total weight of from 400mg to 450mg;
- the 25mg strength exhibits a total weight of from 250mg to 281.25mg;
- the 18mg strength exhibits a total weight of from 180mg to 202.5mg; and
- the 10mg strength exhibits a total weight of from 100mg to 112.5mg with the proviso that the 40mg strength does not exhibit a higher total weight than the 100mg strength.

8. A plurality of 7 dosage forms according to claim 5, wherein
- the first reference strength comprises 100mg Atomoxetine;
- dosage forms comprising from 40mg to 80mg Atomoxetine are weight proportional compared to the first reference strength which contains 100mg of Atomoxetine, and
- the second reference strength comprises 25mg Atomoxetine; and
- dosage forms comprising from 10mg to 18mg Atomoxetine are weight proportional compared to the second reference strength.

9. The plurality of dosage forms according to claim 8, wherein
- the strengths are 10mg, 18mg, 25mg, 40mg, 60mg, 80mg and 100mg,
- the active substance is Atomoxetine in the form of its hydrochloride salt;
- the 100mg strength exhibits the same total weight of from 250mg to 450mg;
- the 80mg strength exhibits a total weight of from 200mg to 360mg;
- the 60mg strength exhibits a total weight of from 150mg to 270mg;
- the 40mg strength exhibits a total weight of from 100mg to 180mg;
- the 25mg strength exhibits a total weight of from 250mg to 450mg;
- the 18mg strength exhibits a total weight of from 180mg to 324mg; and
- the 10mg strength exhibits a total weight of from 100mg to 180mg
with the proviso that the 25mg strength does not exhibit a higher total weight than the 100mg strength.

10. A plurality of 7 dosage forms according to claim 5, wherein
- the first reference strength comprises 100mg Atomoxetine;
- dosage forms comprising from 25mg to 80mg Atomoxetine are weight proportional compared to the first reference strength;
- the second reference strength comprises 18mg Atomoxetine; and
- the dosage form comprising 10mg Atomoxetine is weight proportional compared to the second reference strength.

11. The plurality of dosage forms according to claim 10, wherein
- the strengths are 10mg, 18mg, 25mg, 40mg, 60mg, 80mg and 100mg;
- the active substance is Atomoxetine in the form of its hydrochloride salt;
- the 100mg strength exhibits the same total weight of from 400mg to 450mg;
- the 80mg strength exhibits a total weight of from 320mg to 360mg;
- the 60mg strength exhibits a total weight of from 240mg to 270mg;
- the 40mg strength exhibits a total weight of from 160mg to 180mg;
- the 25mg strength exhibits a total weight of from 100mg to 112.5;
- the 18mg strength exhibits a total weight of from 180mg to 450mg; and
- the 10mg strength exhibits a total weight of from 100mg to 250mg
with the proviso that the 18mg strength does not exhibit a higher total weight than the 100mg strength.

12. The plurality of dosage forms according to one of the previous claims, wherein the dosage form is a film coated tablet.

13. The plurality of film coated tablets according to claim 12, comprising from 25% to 60% by weight of one or more fitter(s);

14. The plurality of film coated tablets according to claim 13, further comprising
- from 25% to 40% by weight of one or more binder(s);
- from 1 % to 10% by weight of one or more disintegrant(s);
- from 1 % to 5% by weight of one or more glidant(s);
- from 0.5% to 3% by weight of one or more lubricant(s); and
- optionally one or more other pharmaceutically acceptable excipient(s).

15. A process for the manufacture of a plurality of immediate release pharmaceutical dosage forms according to claim 14, comprising the following steps:
(a) The drug substance, fitter(s), binder(s), glidant(s) and portions of disintegrant(s) are weighed, sieved (0.80mm - 1.0mm mesh width) and mixed to form mixture I
(b) lubricant(s) and portion of disintegrant are weighed, sieved (0.5mm mesh width), combined with mixture I and mixed to form the final blend
(c) the final blend is compressed into tablets
(d) the tablets are film coated.
